(19) Europäisches Patentamt
European Patent Office
Office européen des brevets

(11) Veröffentlichungsnummer: **0 075 849**
**B1**

(12) **EUROPÄISCHE PATENTSCHRIFT**

(45) Veröffentlichungstag der Patentschrift: **22.07.87**

(51) Int. Cl.⁴: **C 07 D 401/12**, C 08 K 5/34

(21) Anmeldenummer: **82108750.9**

(22) Anmeldetag: **22.09.82**

(54) **Triazinyllactame, Verfahren zu ihrer Herstellung, ihre Verwendung als Stabilisatoren für synthetische organische Polymere und die mit ihnen stabilisierten Polymeren.**

(30) Priorität: **26.09.81 DE 3138343**

(43) Veröffentlichungstag der Anmeldung: **06.04.83 Patentblatt 83/14**

(45) Bekanntmachung des Hinweises auf die Patenterteilung: **22.07.87 Patentblatt 87/30**

(84) Benannte Vertragsstaaten: **AT BE CH DE FR GB IT LI NL SE**

(56) Entgegenhaltungen: **EP-A-0 024 338**
**US-A-3 417 079**

(73) Patentinhaber: **HOECHST AKTIENGESELLSCHAFT, Postfach 80 03 20, D-6230 Frankfurt am Main 80 (DE)**

(72) Erfinder: **Wiezer, Hartmut, Dr., Am Stocket 18, D-8901 Lützelburg (DE)**
Erfinder: **Pfahler, Gerhard, Dr., Karlsbader Strasse 27, D-8900 Augsburg (DE)**

**EP 0 075 849 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

In der Literatur sind niedermolekulare, piperidingruppenhaltige Triazinvorbindungen als Stabilisatoren für Polymere beschrieben (z. B. DE-A-23 19 816). Trotz ihrer guten stabilisierenden Wirkung besitzen diese Verbindungen jedoch diverse Nachteile, insbesondere den Nachteil der zu hohen Flüchtigkeit. Eine Verbesserung oder sogar das Ausmerzen dieser nachteiligen Eigenschaften wurde in der Synthese hochmolekularer Stabilisatoren gesehen (z. B. DE-A-29 44 729; DE-C-26 36 144; EP-A 13 635, DE-A-27 19 131). Hierbei sind jedoch verfahrenstechnische Probleme, etwa die des Einstellens der gewünschten Molekulargewichte, nur unter großen Schwierigkeiten zu beheben. Es hat daher nicht an Versuchen gefehlt, Produkte definierten Molgewichts und niedriger Flüchtigkeit herzustellen, wobei die Molgewichte solcher Verbindungen im Bereich von ca. 2000 liegen. Aber auch diese Verbindungen besitzen noch eine zum Teil erhebliche Flüchtigkeit, wofür wahrscheinlich verfahrensbedingte niedrigmolekulere Anteile verantwortlich sind.

Neben den oben zitierten niedrigmolekularen Triazinverbindungen sind zahlreiche weitere niedrigmolekulare Stabilisatoren für Polymere auf Basis sterisch gehinderter Amine bekannt. Zu den besten dieser Art zählen die Lactamverbindungen der DE-A-26 06 026 und der DE-C-26 34 957; dennoch entsprechen mittlerweile auch diese Produkte bezüglich ihrer Flüchtigkeit und ihres Migrationsverhaltens nicht im vollen Umfang den gestiegenen Anforderungen.

In jüngster Zeit konzentrierten sich die Entwicklungsarbeiten ausschließlich auf hochmolekulare Stabilisatoren, weil man annahm, daß nur solche Produkte die Erwartungen hinsichtlich Flüchtigkeit und Migrationsfestigkeit würden erfüllen können. Es besteht somit ein Vorurteil gegen niedrigmolekulare Stabilisatoren.

Es wurde gefunden, daß neue niedermolekulare Polyalkylpiperidyl- und zusätzlich Lactamgruppen enthaltende Triazinverbindungen überraschenderweise im Vergleich zu den bisherigen Entwicklungen niedrigen Molgewichts neben hervorragender, stabilisierender Wirksamkeit auch eine sehr geringe Flüchtigkeit besitzen.

Die neuen Triazinyllactame entsprechen der allgemeinen Formel (I),

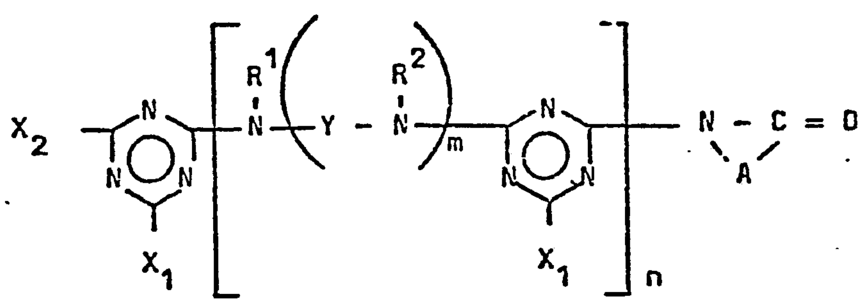

(I),

in welcher

m 0 oder 1, vorzugsweise 1,

n 0 oder 1 und

A = Alkylen mit 4 bis 11, vorzugsweise 5 bis 11, und insbesondere 5 C-Atomen ist.

$R^1$ hat bei m = 0 die Bedeutung von Wasserstoff, von $C_1$- bis $C_{18}$-, vorzugsweise $C_1$- bis $C_{12}$- und insbesondere $C_1$- bis $C_8$-Alkyl, von $C_3$- bis $C_{21}$-Alkoxialkyl, vorzugsweise $C_1$- bis $C_{18}$-Alkoxipropyl, insbesondere $C_1$- oder $C_2$-Alkoxipropyl, von $C_5$- bis $C_{12}$-Cycloalkyl-, vorzugsweise Cyclohexylresten, die durch $C_1$- bis $C_4$-Alkyl 1 bis 4fach, vorzugsweise 1fach substituiert sein können, von Phenyl oder Naphthyl, das durch bis zu zwei Cl, durch bis zu zwei $C_1$- bis $C_4$-Alkylgruppen, vorzugsweise Methyl, durch eine $C_1$- bis $C_4$-, vorzugsweise $C_1$-Alkoxigruppe, oder durch eine $C_1$- oder $C_2$-Carbalkoxigruppe substituiert sein kann, vorzugsweise jedoch Phenyl, von $C_7$- bis $C_{14}$-, vorzugsweise $C_7$- bis $C_9$-Phenylalkyl, oder einer Gruppe der Formel (II),

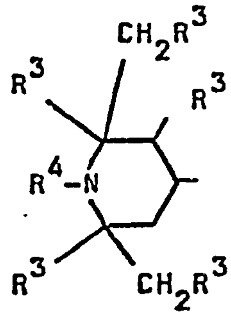

(II)

in welcher
$R^3$ Wasserstoff oder Methyl, vorzugsweise Wasserstoff und
$R^4$ Wasserstoff, $C_1$- bis $C_4$-Alkyl, 2,5-Epoxipropyl, Allyl oder Benzyl, vorzugsweise jedoch Wasserstoff ist.

$R^1$ und $R^2$ sind bei $m = 1$ gleich oder verschieden und stehen für Wasserstoff, für $C_1$- bis $C_{18}$-, vorzugsweise $C_1$- bis $C_{12}$ -Alkyl, für vorzugsweise unsubstituiertes $C_5$- bis $C_{12}$-Cycloalkyl, das auch durch bis zu vier $C_1$- bis $C_4$-, insbesondere Methylgruppen substituiert sein kann, für Phenyl, das durch bis zu zwei $C_1$- bis $C_4$-Alkyl-reste substituiert sein kann, für $C_7$- bis $C_{14}$-, vorzugsweise $C_7$- bis $C_9$-Phenylalkyl oder für die Gruppe der Formel (II).

$X_1$ und $X_2$ sind bei $n = 0$ gleich oder verschieden und bedeuten Halogen, vorzugsweise Chlor, Phenyl oder eine Gruppe der Formel (III), (IV) oder (V), vorzugsweise (III) oder (IV) und insbesondere (III),

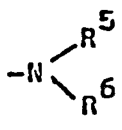

(III),

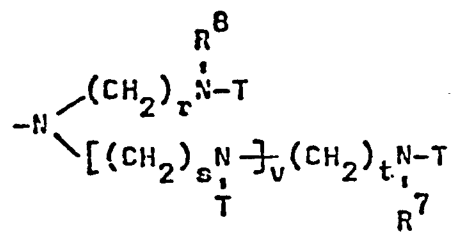

(IV),

$-OR^9$ (V).

Bei $n = 1$ ist $X_2$ ausschließlich eine Gruppe der Formel

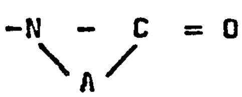

mit A = $C_4$- bis $C_{11}$-, vorzugsweise $C_5$- bis $C_{11}$- und insbesondere $C_5$ -Alkylen.

In Formel (III) sind
$R^5$ und $R^6$ gleich oder verschieden und haben die für $R^2$ angegebene Bedeutung und
$R^6$ hat zusätzlich die Bedeutung von $C_3$- bis $C_{21}$-Alkoxialkyl, vorzugsweise $C_1$- bis $C_{18}$-Alkoxipropyl und insbesondere $C_1$- oder $C_2$-Alkoxipropyl.

$R^5$ und $R^6$ können ferner zusammen mit dem sie bindenden N-Atom für einen Pyrrolidin- oder einen unsubstituierten oder durch bis zu vier $C_1$- bis $C_4$-Alkylgruppen, vorzugsweise Methylgruppen substituierten Piperidin-, Morpholin- oder Hexamethyleniminring stehen.

In Formel (IV) bedeuten die Indices
r, s und t gleiche oder unterschiedliche ganze Zahlen von 2 bis 6, vorzugsweise 2 oder 3,
v eine ganze Zahl von 0 bis 3, vorzugsweise 0 oder 1 und insbesondere 0, und stehen
$R^7$ und $R^8$ für gleiche oder verschiedene Reste mit den fü $R^2$ angegebenen Bedeutungen, vorzugsweise für Wasserstoff oder eine Gruppe der Formel (II) und insbesondere für Wasserstoff und hat
T die Bedeutung einer Gruppe der Formel (VI)

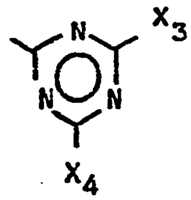

(VI),

in welcher

$X_3$ und $X_4$ gleiche oder verschiedene Reste der Formel (III), (IV) oder (V), vorzugsweise (III) oder (IV) und insbesondere (III) sind.

In Formel (V) steht

$R^9$ für einen der unter $R^1$ bei m = 0 aufgeführten Reste.

Y ist $C_2$- bis $C_{18}$-, vorzugsweise $C_2$- bis $C_{12}$- und insbesondere $C_2$- bis $C_6$-Alkylen, oder $C_2$- bis $C_{12}$-Bis-(propyloxi)-alkylen, oder Mono-, Di- oder Tri-Cycloalkylen mit 6 bis 18, vorzugsweise 6 bis 12 C-Atomen, das durch bis zu vier Methylgruppen substituiert sein kann, vorzugsweise jedoch ein unsubstituiertes, wobei bis zu zwei C-Atome durch N-Atome, die Propylengruppen tragen können, ersetzt sein können, oder $C_6$- bis $C_{18}$-Arylen, vorzugsweise Phenylen, oder $C_7$- bis $C_{18}$-Aralkylen. An die Stelle der Gruppierung

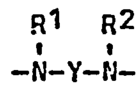

können auch Piperazinylen-Reste oder an Stelle der Gruppierungen

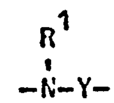

oder

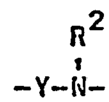

durch bis zu vier Methylengruppen substituierte $C_5$- bis $C_9$-Aza-Monocyclo- oder Bicycloalkylenreste treten.

In Formel (I) muß mindestens eine, bevorzugt pro Triazinring mindestens eine Gruppe der Formel (II) enthalten sein.

Die neuen Triazinyllactame werden durch Umsetzen von Verbindungen der Formel (VII)

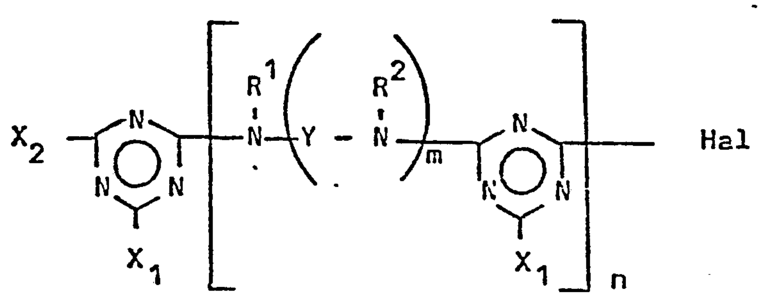

(VII),

in der $R^1$, $R^2$, $X_1$, $X_2$, Y, m und n die vorstehend angegebenen Bedeutungen haben und Hal vorzugsweise Chlor ist, mit der 1,0- bis 1,05fach-valenten, vorzugsweise äquivalenten Menge, bezogen auf die zur Umsetzung bestimmten Halogenatome, von Lactammetallsalzen, insbesondere Natrium- oder Kalium-Lactamaten bei Temperaturen von 100 bis 200, vorzugsweise 130 bis 200 und insbesondere 160 bis 200°C hergestellt.

Als Lösungsmittel sind inerte organische Solventien wie Toluol, Xylol, Mesitylen, Dioxan etc. geeignet. Falls erforderlich, können die Reaktionen auch unter Druck durchgeführt werden.

Als Beispiele für Verbindungen der Formel (VI), die aus Cyanurhalogenid und Aminen z. B. nach DE-A-23 19 816, 29 44 729, 30 45 839 und 31 13 455 zugänglich sind, seien genannt:

1. 2-Chlor-4,6-bis-[N-(2,2,6,6-tetramethyl-4-piperidinyl)-3-methoxipropan-1-amino]-1,3,5-triazin
2. 2-Chlor-4,6-bis-[N-(2,2,6,6-tetramethyl-4-piperidinyl)-3-ethoxipropan-1-amino]-1,3,5-triazin
3. 2-Chlor-4,6-bis-[N-(2,2,6,6-tetramethyl-4-piperidinyl)-3-dimethylaminopropan-1-amino]-1,3,5-triazin
4. 2-Chlor-4,6-bis-[N-(2,2,6,6-tetramethyl-4-piperidinyl)-butylamino]-1,3,5-triazin
5. 2-Chlor-4,6-bis-[N-(2,2,6,6-tetramethyl-4-piperidinyl)-octa-decylamino]-1,3,5-triazin

6. 2-Chlor-4,6-bis-[N-(2,2,6,6-tetramethyl-4-piperidinyl)-amino]-1,3,5-triazin
7. 2-Chlor-4,6-bis-[N-(2,2,6,6-tetramethyl-4-piperidinyl)-cyclohexylamino]-1,3,5-triazin
8. 2-Chlor-4,6-bis-[N-(2,2,6,6-tetramethyl-4-piperidinyl)-hexylamino]-1,3,5-triazin
9. 2-Chlor-4-[N-(2,2,6,6-tetramethyl-4-piperidinyl)-3-ethoxi-propylamino]-6-dicyclohexylamino-1,3,5-triazin
10. 2-Chlor-4-[N-(2,2,6,6-tetramethyl-4-piperidinyl)-3-methoxi-propan1-amino]-6-dioctadecylamino-1,3,5-triazin
11. 2-Chlor-4,6-{1,9-bis-[2.4-bis-<N-(2,2,6,6-tetramethyl-4-piperidinyl)-3-methoxipropan-1-amino>-1,3,5-triazin-6-yl]-1,5,9,triazanonyl-5-}-1,3,5-triazin
12. 3-Methoxipropyl-bis-[2-chlor-4-<N-(2,2,6,6-tetramethyl-4-piperidinyl)-3-methoxi-propan-1-amino>-1,3,5-triazin-6-yl-imid
13. Butyl-bis-[2-chlor-4-<N-(2,2,6,6-tetramethyl-4-piperidinyl)-butylamino>-1,3,5-triazin-6-yl]-imid
14. N,N'-Bis-[2-chlor-4-<N-(2,2,6,6-tetramethyl-4-piperidinyl)-butylamino>-1,3,5-triazin-6-yl]-1,6-hexandiamin
15. N,N'-Bis-[2-chlor-4-<N-(2,2,6,6-tetramethyl-4-piperidinyl)-5-methoxipropan-1-amino>-1,3,5-triazin-6-yl]-N,N'-bis-(2,2,6,6-tetramethyl-4-piperidinyl)-3(4),8(9)-bis-(amino-ethyl)-tricyclo-[5.2.1.$0^{2,6}$]-decan-Isomerengemisch

Unter Lactamsalzen werden bevorzugt die Kalium- und Natriumlactamate, insbesondere die letztgenannten, die aus Lactamen und Natriumhydrid zugänglich sind, verstanden. Es kommen die Lactamate der Lactame mit 4 bis 11, vorzugsweise 5 bis 11 und insbesondere 5 Methylengruppen zum Einsatz.

Die neuen Verbindungen besitzen - wie bereits erwähnt - eine außerordentlich niedrige Flüchtigkeit, die mit der der Stabilisatoren nach DE-A-29 44 729 vergleichbar ist, was aufgrund der dargelegten Vorurteile gegen niedrigmolekulare Verbindungen nicht erwartet werden konnte, und daher als überraschend angesehen werden muß. Sie eignen sich daher in besonderem Maße als Stabilisatoren für Kunststoffe gegen deren Schädigung durch die Einwirkung von Sauerstoff, Wärme und Licht. Beispiele für solche Kunststoffe, für Additive, die als weitere Zusatzstoffe eingesetzt werden sowie Stabilisierbeispiele sind in der DE-A-31 13 455, Seiten 19 bis 24, aufgeführt.

Insbesondere eignen sich die neuen Triazinyllactame zum Stabilisieron von Polyolefinen, Polystyrol und Polyacrylaten.

In den folgenden Beispielen, die der weiteren Erläuterung des Erfindungsgegenstandes dienen, sind die eingesetzten Halogentriazine durch Ziffern gekennzeichnet, welche sich auf die auf den Seiten 7 und 8 aufgelisteten Verbindungen beziehen. Die Produkte stellen sämtlich harzartige Substanzen dar, welche naturgemäß keine scharfen Schmelzpunkte zeigen. Ist ein Produkt als "Harz" bezeichnet, so handelt es sich um ein Weichharz.

**Beispiel 1**

In einer 250-ml-Rührapparatur werden 6,75 g (0,05 Mol) ε-Caprolactam-Natriumsalz und 28,3 g (0,05 Mol) der Verbindung Nr. 1 in 150 ml wasserfreiem Mesitylen 20 h bei 140° C gerührt. Ausgefallenes NaCl wird abfiltriert, das Filtrat mit Aktivkohle/ Bleicherde 1 h gekocht, filtriert und im Vakuum zur Trockene eingedampft. Es bleiben 32,0 g eines gelblichen Harzes vom Fp. 65 bis 70° C zurück. Molgewicht 644.

IR: $\gamma_{C=0}$ bei 1700 $cm^{-1}$ (zum Vergleich $\gamma_{C=0}$ des ε-Caprolactams liegt bei 1661 $cm^{-1}$)

**Beispiele 2 bis 8**

Analog Beispiel 1 wurden unter Einsatz anderer Na-Lactamate und Chlortriazine hergestellt:

| Bsp.Nr. | Chlortriazin (g = Mol) | Na-Lactamat (g = Mol) | $\gamma_{C=0}$ ($cm^{-1}$) | Fp (°C) |
|---|---|---|---|---|
| 2 | Verb.-Nr. 2 (29,8 = 0,05) | wie in Bsp. 1 | 1699 | Harz |
| 3 | Verb.-Nr. 9 (29,6 = 0,05) | dto. | 1700 | dto. |
| 4 | Verb.-Nr. 4 (26,8 = 0,05) | dto. | 1700 | dto. |
| 5 | Verb.-Nr. 10 (26,8 = 0,05) | dto. | 1700 | 53-66 |
| 6 | Verb.-Nr. 11 (43,0 = 0,05) | dto. | 1700 | Harz |
| 7 | Verb.-Nr. 15 (28,8 = 0,025) | dto. | 1700 | 100-30 |
| 8 | Verb.-Nr. 1 (28,3 = 0,05) | Na-ω-Laurinlactamat +) (10,95 = 0,05) | 1670 | 118-40 |

+) $\gamma_{C=0}$ ω-Laurinlactam 1635 $cm^{-1}$

**Beispiel 9**

Dieses Beispiel zeigt die Flüchtigkeit der neuen Triazinstabilisatoren im Vergleich zu einen Produkt des nächsten Standes der Technik.

Die Flüchtigkeiten wurden in einer Apparatur zur thermogravimetrischen Analyse bestimmt. Jeweils die gleiche Menge (500 mg) der erfindungsgemäßen Verbindungen und der Vergleichssubstanzen wurde dazu in Stickstoffatmosphäre mit einer Aufheizgeschwindigkeit von 2 K/min bis auf 300°C erhitzt und der Substanzverlust in mg/cm² Probenoberfläche gemessen. Die Ergebnisse zeigt nachstehende Tabelle:

| Stabilisator gem. Bsp. | Gewichtsverlust in mg/cm² beim Erreichen von ...°C | | | | |
|---|---|---|---|---|---|
| | 220 | 260 | 300 | 10 min bei 300°C | 30 min bei 300°C |
| 1 | 0,16 | 1,42 | 6,64 | 9,80 | 14,22 |
| 8 | 0,32 | 3,32 | 8,69 | 11,69 | 16,91 |
| Vergl.substanz 1 | 1,27 | 2,53 | 11,06 | 19,9 | 39,8 |
| Vergl.substanz 2 | 0,16 | 2,05 | 5,37 | 8,06 | - |
| Vergl.substanz 3 | 1,11 | 1,58 | 4,74 | 6,46 | 9,80 |
| Vergl.substanz 4 | 1,27 | 4,58 | 20,67 | - | - |
| Vergl.substanz 5 | 0,0 | 1,11 | 9,48 | 58,46 | - |

**Vergleichssubstanzen**

Nr. 1: Edukt vom Bsp. 1 (Molgewicht 567,5)

Nr. 2: Verb. gem. Bsp. 16 der DE-A-29 44 729

Nr. 3: N,N',N'', N'''-Tetrakis-[2,4-<N-(2,2,6,6-tetramethyl-4-piperidinyl)-5-methoxi-propan-1-amino>-1,3,5-triazin-6-yl]-N,N'-1,2-ethandiylbis-1,3-propandiamin (Verb. aus DE-A-29 44 720; Molgewicht: ca. 2300)

Nr. 4: Verb. gem. Bsp. 31 der DE-A-26 06 026 (Molgewicht: 364)

Nr. 5: Polymeres gem. Bsp. 1 der DE-A-27 19 131 (Handelsprodukt Tinuvin 622)

**Beispiel 10**

Aus 100 Gew.-Teilen Polypropylen (® Hostalen PPK 0160 der Hoechst AG) vom Schmelzindex MFI 190/5 = 1,9 g/10 min s. DIN 53 535),

0,2 Gew.-Teilen Calciumstearat,

0,1 Gew.-Teilen Pentaerythrityl-tetrakis-3-(3,5-di-t.-butyl-4-hydroxyphenyl)-propionat und

0,3 Gew.-Teilen des zu prüfenden Stabilisators

werden in einem Laborschnellmischer Mischungen hergestellt, die man zu Granulat verarbeitet, worauf man das so vorbereitete Material in einem Laborextruder unter den üblichen Verarbeitungsbedingungen aufschmilzt und über eine Spinnpumpe mit Achtfachspinnkopf zu Monofilamanten verspinnt, welche anschließend im Verhältnis 1:3 nachverstreckt, zu Garn von 40 dtex texturiert und zu Prüfgeweben vorarbeitet werden. Die Gewebeproben werden sodann auf einen gelochten Rahmen so aufgespannt, daß eine freie Öffnung von ca. 15,5 mm Durchmesser bestehen bleibt. In dieser Form werden die Prüflinge in einem Xenotest X 1200-Gerät der Firma Original Hanau Quarzlampen GmbH unter Bestrahlung mit Wechsellicht künstlich bewittert. Die Strahlungsintensität wurde durch UV-Filter (Spezialfilterglas d = 1,7 mm) moduliert. Die Lichtbeständigkeit wurde nach DIN 53 387 (17 min Trockenperiode, 3 min beregnen, Schwarztafeltemperatur 45°C, relative Luftfeuchtigkeit während der Trockenperiode 70 bis 75 %) geprüft. In bestimmten Zeitabständen werden die Gewebe zentrisch mit einem Gewicht von 6 mm Durchmesser und einem Druck von 0,1 $N/m^2$ belastet. Als Prüfendpunkt gilt das Durchbrechen des Gewichts.

| Stabilisator nach Beispiel | Belichtungszeit in h |
|---|---|
| 1 | > 2000 ++) |
| 8 | > 2000 ++) |
| Polypropylen +) | 300 |
| Vergleichssubstanz 1 | 1250 |
| Vergleichssubstanz 3 | > 2000 ++) |
| Vergleichssubstanz 6 | 1750 |

+) ohne Lichtschutzstabilisator
++) nach 2 000 Stunden Gewebe noch in Ordnung

**Vergleichssubstanzen**

Nr. 1, 3: wie im Beispiel 9
Nr. 6: Produkt gemäß DE-A-23 19 816 der Formel

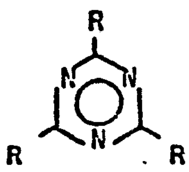

R =

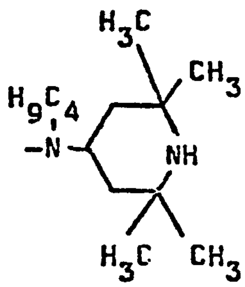

**Patentansprüche**

für die Vertragsstaaten BE, CH, DE, FR, GB, IT, LI, NL, SE.

1. Verbindungen der allgemeinen Formel I

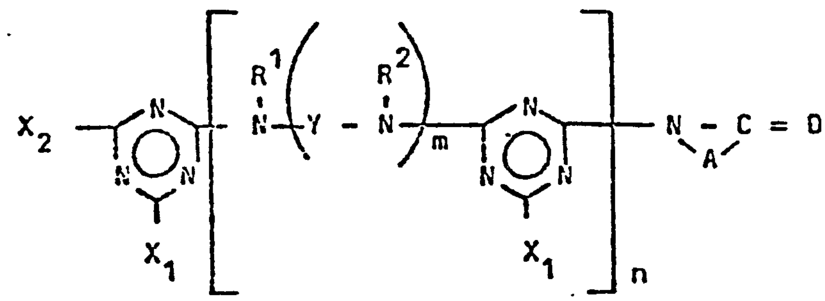

(I)

in welcher
m 0 oder 1
n ebenfalls 0 oder 1 und
A $C_4$- bis $C_{11}$-Alkylen ist,

$R^1$ bei m = 0 die Bedeutung von Wasserstoff, von $C_1$- bis $C_{18}$-Alkyl, von $C_3$- bis $C_{21}$-Alkoxialkyl, von $C_5$- bis $C_{12}$-Cycloalkyl, das durch $C_1$- bis $C_4$-Alkyl substituiert sein kann, von Phenyl oder Naphthyl, das durch bis zu zwei Cl oder $C_1$- bis $C_4$-Alkylreste oder durch eine $C_1$- bis $C_4$-Alkoxi- oder $C_1$ - oder $C_2$-Carbalkoxi-gruppe substituiert sein kann, von $C_7$- bis $C_{14}$-Phenylalkyl oder einer Gruppe der Formel (II)

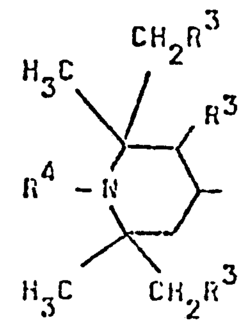

(II)

hat, worin
$R^3$ Wasserstoff oder Methyl und
$R^4$ Wasserstoff, $C_1$- bis $C_4$-Alkyl, 2,3-Epoxipropyl, Allyl oder Benzyl ist,

$R^1$ und $R^2$ bei m = 1 gleich oder verschieden sind und Wasserstoff, $C_1$- bis $C_{18}$-Alkyl, $C_5$- bis $C_{12}$-Cycloalkyl, das durch bis zu vier $C_1$- bis $C_4$-Alkylgruppen substituiert sein kann, Phenyl, das durch bis zu zwei $C_1$- bis $C_4$-Alkylreste substituiert sein kann, $C_7$- bis $C_{14}$-Phenylalkyl oder eine Gruppe der Formel (II) darstellen,

$X_1$ und $X_2$ bei n = 0 gleich oder verschieden sind und für Halogen, für Phenyl oder für eine Gruppe der Formeln (III), (IV) oder (V)

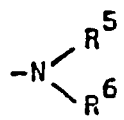

(III),

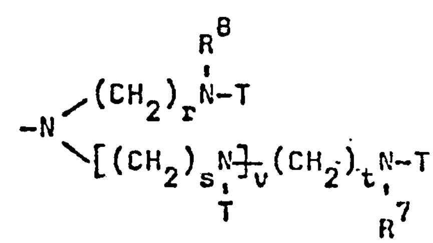

(IV)

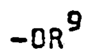

(V)

stehen, jedoch
bei n = 1 $X_2$ ausschließlich eine Gruppe der Formel

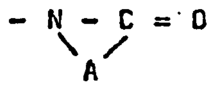

mit A = $C_4$- bis $C_{11}$-Alkylen ist, wobei in den Formeln (III) (IV) und (V)
$R^5$ und $R^6$ gleiche oder verschiedene, für aufgeführte Reste darstellen und
$R^6$ zusätzlich die Bedeutung von $C_3$- bis $C_{21}$-Alkoxialkyl,
hat, oder
$R^5$ und $R^6$ auch zusammen mit dem sie bindenden N-Atom für einen Pyrrolidin- oder einen unsubstituierten oder durch bis zu vier $C_1$- bis $C_4$-Alkylgruppen substituierten Piperidin-, Morpholin- oder Hexamethyleniminring stehen,
r, s und t gleiche oder unterschiedliche ganze Zahlen von 2 bis 6 und
v von 0 bis 3 sind,
$R^7$ und $R^8$ gleiche oder verschiedene Reste mit den für $R^2$ angegebenen Bedeutungen darstellen,
T die Bedeutung einer Gruppe der Formel (VI)

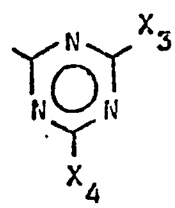

(VI)

hat, mit $X_3$ und $X_4$ = gleiche oder verschiedene Reste der Formeln (III), (IV) oder (V),
$R^9$ einer der unter $R^1$ bei m = 0 genannten Reste ist, und
Y für $C_2$- bis $C_{18}$-Alkylen, für $C_2$- bis $C_{12}$-Bis-(propyloxi)-alkylen, für Mono-, Di- oder Tricycloalkylen mit 6 bis 18 C-Atomen, das durch bis zu vier Methylgruppen substituiert sein kann, in welchem bis zu zwei C-Atome durch N-Atome, die Propylengruppen tragen können, ersetzt sein können, für $C_6$- bis $C_{18}$-Arylen, oder für $C_7$- bis $C_{14}$ -Aralkylen steht, oder auch an Stelle der Gruppierung

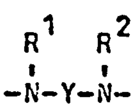

ein Piperazinylenrest oder an Stelle der Gruppierungen

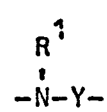

oder

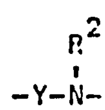

durch bis zu vier Methylgruppen substituierte $C_5$- bis $C_9$-Aza-Monocyclo- oder -Dicycloalkylenreste treten,
und in Formel (I) mindestens eine, bevorzugt pro Triazinring mindestens eine Gruppe der Formel (II) enthalten ist.

2. Verfahren zur Herstellung der Verbindungen nach Anspruch 1, dadurch gekennzeichnet, daß man eine Verbindung der Formel (VII)

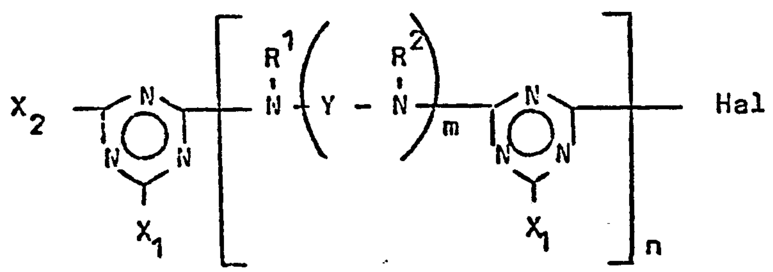

(VII)

in der $R^1$, $R^2$, $X_1$, $X_2$, Y, m und n die in Anspruch 1 angegebenen Bedeutungen haben, und Hal vorzugsweise Chlor ist, in einem inerten organischen Solvens bei 100 bis 200°C mit der, bezogen auf die zur Umsetzung bestimmten Halogenatome, 1,0- bis 1,05fach valenten Menge des Alkalimetallsalzes eines Lactams mit 4 bis 11 Methylengruppen umsetzt.

3. Verwendung der Verbindungen nach Anspruch 1 zum Stabilisieren von synthetischen Polymeren.

4. Verwendung nach Anspruch 3, dadurch gekennzeichnet, daß das Polymere ein Polyolefin, Polyacrylat, Polymethacrylat oder ein Homo- odor Copolymerisat des Styrols ist.

5. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen 0,01 bis 5 Gewichtsteile, bezogen auf Polymeres, eines Stabilisators nach Anspruch 1 zusetzt.

6. Gegen UV-Zersetzung stabilisierte, synthetische Polymere, in denen 0,01 bis 5 Gewichtsteile, bezogen auf Polymeres, eines Stabilisators nach Anspruch 1 enthalten sind.

**Patentansprüche**

für den Vertragsstaat Österreich

1. Verfahren zur Herstellung von Triazinyllactamen der allgemeinen Formel (I)

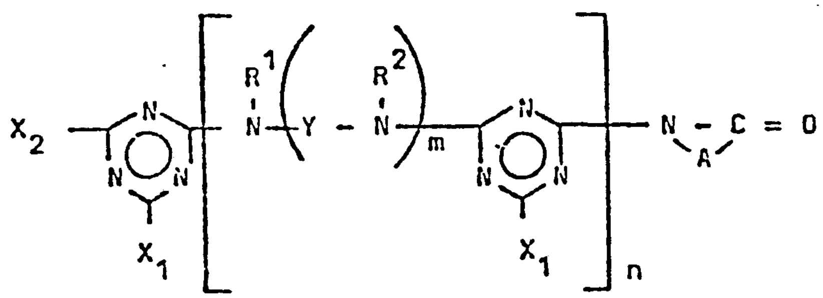

(I)

in welcher
m 0 oder 1,
n ebenfalls 0 oder 1 und
A $C_4$- bis $C_{11}$-Alkylen ist,
$R^1$ bei m = 0 die Bedeutung von Wasserstoff, von $C_1$- bis $C_{18}$-Alkyl, von $C_3$- bis $C_{21}$-Alkoxialkyl, von $C_5$- bis $C_{12}$-Cycloalkyl, das durch $C_1$- bis $C_4$-Alkyl substituiert sein kann, von Phenyl oder Naphthyl, das durch bis zu zwei Cl oder $C_1$- bis $C_4$-Alkylreste oder durch eine $C_1$- bis $C_4$-Alkoxi- oder $C_1$- oder $C_2$-Carbalkoxi-gruppe substituiert sein kann, von $C_7$- bis $C_{14}$-Phenylalkyl oder einer Gruppe der Formel (II)

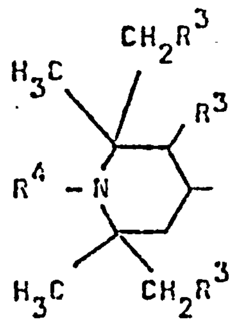

(II)

hat, worin
$R^3$ Wasserstoff oder Methyl und
$R^4$ Wasserstoff, $C_1$- bis $C_4$-Alkyl, 2,3-Epoxipropyl, Allyl oder Benzyl ist,
$R^1$ und $R^2$ bei $m = 1$ gleich oder verschieden sind und Wasserstoff, $C_1$- bis $C_{18}$-Alkyl, $C_5$- bis $C_{12}$-Cycloalkyl, das durch bis zu vier $C_1$- bis $C_4$-Alkylgruppen substituiert sein kann, Phenyl, das durch bis zu zwei $C_1$- bis $C_4$-Alkylreste substituiert sein kann, $C_7$- bis $C_{14}$-Phenylalkyl oder eine Gruppe der Formel (II) darstellen,
$X_1$ und $X_2$ bei $n = 0$ gleich oder verschieden sind und für Halogen, für Phenyl oder für eine Gruppe der Formeln (III), (IV) oder (V)

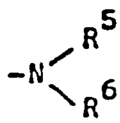 (III), 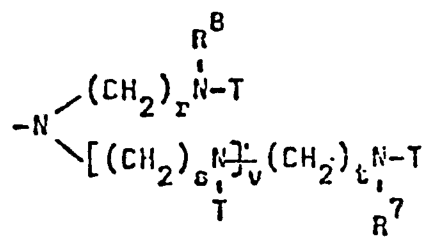 (IV)

$-OR^9$ (V)

stehen, jedoch
bei $n = 1$ $X_2$ ausschließlich eine Gruppe der Formel

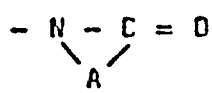

mit A = $C_4$- bis $C_{11}$-Alkylen ist,
wobei in den Formeln (III), (IV) und (V)
$R^5$ und $R^6$ gleiche oder verschiedene, für $R^2$ aufgeführte Reste darstellen und
$R^6$ zusätzlich die Bedeutung von $C_3$- bis $C_{21}$-Alkoxialkyl hat, oder
$R^5$ und $R^6$ auch zusammen mit dem sie bindenden N-Atom für einen Pyrrolidin- oder einen unsubstituierten oder durch bis zu vier $C_1$- bis $C_4$-Alkylgruppen substituierten Piperidin-, Morpholin- oder Hexamethyleniminring stehen,
r, s und t gleiche oder unterschiedliche ganze Zahlen von 2 bis 6 und
v von 0 bis 3 sind,
$R^7$ und $R^8$ gleiche oder verschiedene Reste mit den für $R^2$ angegebenen Bedeutungen darstellen,
T die Bedeutung einer Gruppe der Formel (VI)

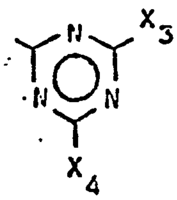

(VI)

hat, mit
$X_3$ und $X_4$ = gleiche oder verschiedene Reste der Formeln (III), (IV) oder (V),
$R^9$ einer der unter $R^1$ bei m = 0 genannten Reste ist, und
Y für $C_2$- bis $C_{18}$-Alkylen, für $C_2$- bis $C_{12}$-Bis-(propyloxi)-alkylen, für Mono-, Di- oder Tricycloalkylen mit 6 bis 18 C-Atomen, das durch bis zu vier Methylgruppen substituiert sein kann, in welchem bis zu zwei C-Atome durch N-Atome, die Propylengruppen tragen können, ersetzt sein können, für $C_6$- bis $C_{18}$-Arylen, oder für $C_7$- bis $C_{14}$-Aralkylen steht, oder auch an Stelle der Gruppierung

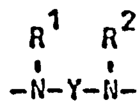

ein Piperazinylenrest oder an Stelle der Gruppierungen

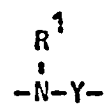

oder

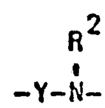

durch bis zu vier Methylgruppen substituierte $C_5$- bis $C_9$-Aza-Monocyclo- oder -Dicycloalkylenreste treten,
und in Formel (I) mindestens eine, bevorzugt pro Triazinring mindestens eine Gruppe der Formel (II) enthalten ist, dadurch gekennzeichnet,
daß man eine Verbindung der Formel (VII)

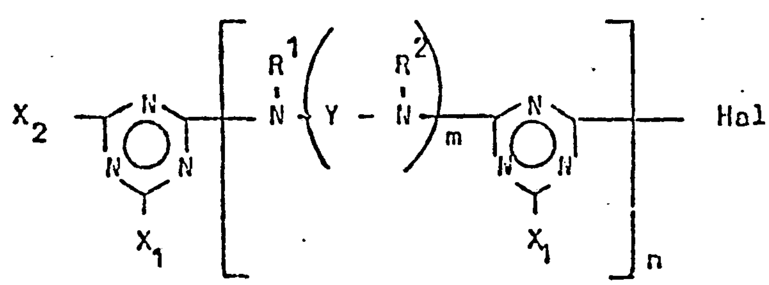

(VII)

in der $R^1$, $R^2$, $X_1$, $X_2$, Y, m und n die oben angegebenen Bedeutungen haben, und Hal vorzugsweise Chlor ist, in einem inerten organischen Solvens bei 100 bis 200°C mit der, bezogen auf die zur Umsetzung bestimmten Halogenatome, 1,0- bis 1,05fach valenten Menge des Alkalimetallsalzes eines Lactams mit 4 bis 11 Methylengruppen umsetzt.

2. Verwendung der Verbindungen nach Anspruch 1 zum Stabilisieren von synthetischen Polymeren.

3. Verwendung nach Anspruch 2, dadurch gekennzeichnet, daß das Polymere ein Polyolefin, Polyacrylat, Polymethacrylat oder ein Homo- oder Copolymerisat des Styrols ist.

4. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen 0,01 bis 5 Gewichtsteile, bezogen auf Polymeres, eines Stabilisators nach Anspruch 1 zusetzt.

5. Gegen UV-Zersetzung stabilisierte, synthetische Polymere, in denen 0,01 bis 5 Gewichtsteile, bezogen auf

Polymeres, eines Stabilisators nach Anspruch 1 enthalten sind.

## Claims

for the Contracting states: BE, CH, DE, FR, GB, IT, LI, NL, SE.

1. A compound of the formula (I)

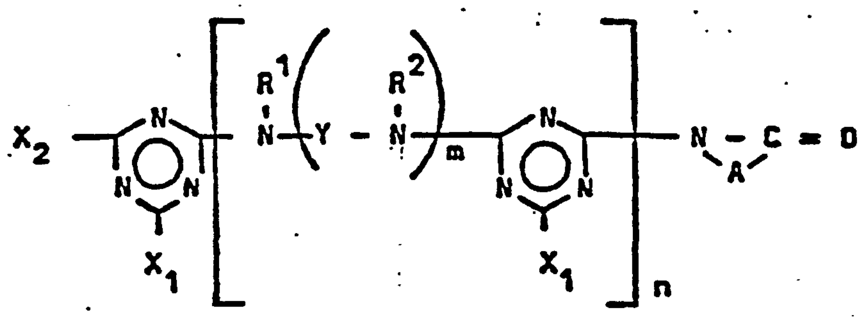 (I)

in which m is 0 or 1, n is also 0 or 1 and A is $C_4$ to $C_{11}$ alkylene, $R^1$, when m is 0, has the meaning of hydrogen, of $C_1$ to $C_{18}$ alkyl, of $C_3$ to $C_{21}$ alkoxyalkyl, of $C_5$ to $C_{12}$ cycloalkyl, which can be substituted by $C_1$ to $C_4$ alkyl, or phenyl or naphthyl, which can be substituted by up to two Cl or $C_1$ to $C_4$ alkyl radicals or by one $C_1$ to $C_4$ alkoxy or $C_1$ to $C_2$ carboalkoxy group, of $C_7$ to $C_{14}$ phenylalkyl or of a group of the formula (II)

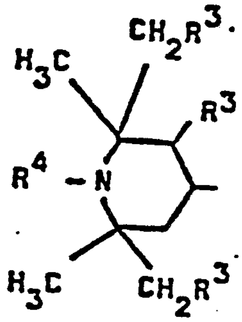 (II)

wherein $R^3$ is hydrogen or methyl and $R^4$ is hydrogen, $C_1$ to $C_4$ alkyl, or is 2,3-epoxypropyl, allyl or benzyl, $R^1$ and $R^2$, when m is 1, are identical or different and represent hydrogen, $C_1$ to $C_{18}$ alkyl, $C_5$ to $C_{12}$ cycloalkyl, which can be substituted by up to four $C_1$ to $C_4$ alkyl groups, phenyl, which can be substituted by up to two $C_1$ to $C_4$ alkyl radicals, $C_7$ to $C_{14}$ phenylalkyl or a group of the formula (II), $X_1$ and $X_2$, when n is 0, are identical or different and represent halogen, phenyl or a group of the formulae (III), (IV) or (V)

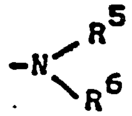 (III), 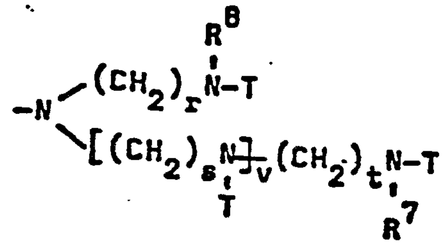 (IV)

$-OR^9$ (V)

but when n is 1, $X_2$ is always a group of the formula

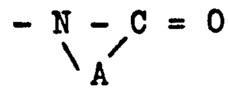

with A being $C_4$ to $C_{11}$ alkylene, wherein, in the formulae (III), (IV) and (V), $R^5$ and $R^6$ are identical or different and represent radicals listed for $R^2$ and $R^6$ additionally has the meaning of $C_3$ to $C_{21}$ alkoxyalkyl, or $R^5$ and $R^6$, together with the N atom connecting them, also represent a pyrrolidine ring or a piperidine, morpholine or hexamethyleneimine ring which is unsubstituted or substituted by up to four $C_1$ to $C_4$ alkyl groups, r, s, and t are identical or different whole numbers from 2 to 6 and v is from 0 to 3, $R^7$ and $R^8$ represent identical of different radicals with the meanings indicated for $R^2$, T has the meaning of a group of the formula (VI)

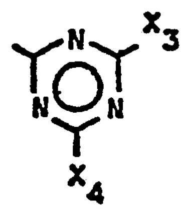

(VI)

with $X_3$ and $X_4$ being identical or different radicals of the formulae (III), (IV) or (V), $R^9$ is one of the radicals mentioned under $R^1$, when m is 0, and Y represents $C_2$ to $C_{18}$ alkylene, $C_2$ to $C_{12}$ bis(propyloxy)alkylene, monocycloalkylene, dicycloalkylene or tricycloalkylene having 6 to 18 C atoms, which can be substituted by up to four methyl groups and in which up to two C atoms can be replaced by N atoms which can carry propylene groups, or Y represents $C_6$ to $C_{18}$ arylene, or $C_7$ to $C_{14}$ aralkylene, or a piperazinylene radical can also take the place of the group

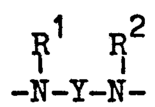 ,

or $C_5$ to $C_6$ azamonocycloalkylene or azadicycloalkylene radicals, substituted by up to four methyl groups, can take the place of the group

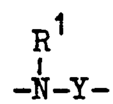

or

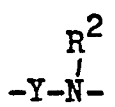 ,

and in formula (I), at least one group of the formula (II) is present, preferably at least one group of the formula (II) per triazine ring.

2. A process for the preparation of a compound as claimed in claim 1, which comprises reacting a compound of the formula (VII)

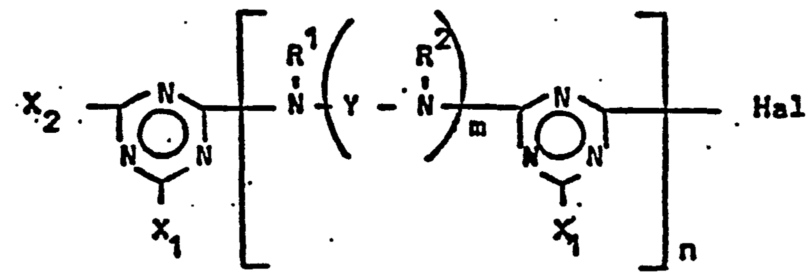

(VII)

in which $R^1$, $R^2$, $X_1$, $X_2$, Y, m and n have the meanings indicated in claim 1 and Hal is preferably chlorine, in an inert organic solvent at 100 to 200°C with 1.0 to 1.05 times the equivalent amount, relative to the halogen atoms envisaged for the reaction, of the alkali metal salt of a lactam having 4 to 11 methylene groups.

3. The use of a compound as claimed in claim 1 for stabilizing synthetic polymers.

4. The use as claimed in claim 3, wherein the polymer is a polyolefin, polyacrylate, polymethacrylate or a homopolymer or copolymer of styrene.

5. A process for stabilizing synthetic polymers against the damaging effects of light, wherein 0.01 to 5 parts by weight, relative to polymer, of a stabilizer as claimed in claim 1 are added to polymers, optionally in addition to substances having a stabilizing action which are already known.

6. A synthetic polymer, stabilized against UV decomposition, containing 0.01 to 5 parts by weight, relative to polymer, of a stabilizer as claimed in claim 1.

## Claims

for the Contracting state: AT

1. A process for the preparation of triazinyllactames of the formula (I)

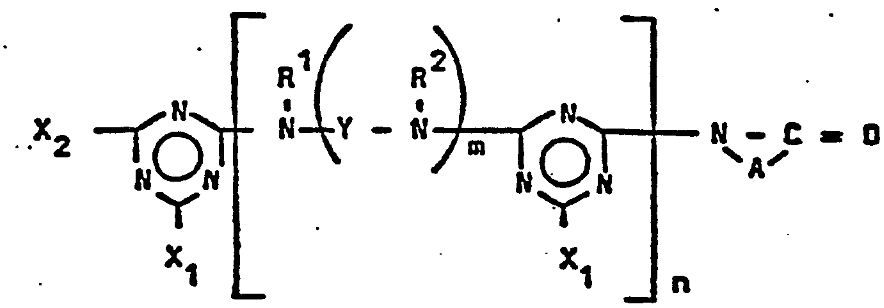

(I)

in which m is 0 or 1, n is also 0 or 1 and A is $C_4$ to $C_{11}$ alkylene, $R^1$, when m is 0, has the meaning of hydrogen, of $C_1$ to $C_{18}$ alkyl, of $C_3$ to $C_{21}$ alkoxyalkyl, of $C_5$ to $C_{12}$ cycloalkyl, which can be substituted by $C_1$ to $C_4$ alkyl, or phenyl or naphthyl, which can be substituted by up to two Cl or $C_1$ to $C_4$ alkyl radicals or by one $C_1$ to $C_4$ alkoxy or $C_1$ to $C_2$ carboalkoxy group, of $C_7$ to $C_{14}$ phenylalkyl or of a group of the formula (II)

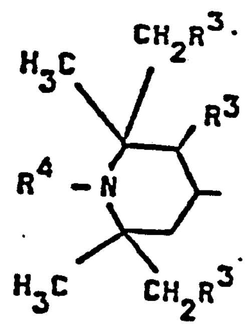

(II)

wherein $R^3$ is hydrogen or methyl and $R^4$ is hydrogen, $C_1$ to $C_4$ alkyl, or is 2,3-epoxypropyl, allyl or benzyl, $R^1$ and $R^2$, when m is 1, are identical or different and represent hydrogen, $C_1$ to $C_{18}$ alkyl, $C_5$ to $C_{12}$ cycloalkyl, which can be substituted by up to four $C_1$ to $C_4$ alkyl groups, phenyl, which can be substituted by up to two $C_1$ to $C_4$ alkyl radicals $C_7$ to $C_{14}$ phenylalkyl or a group of the formula (II), $X_1$ and $X_2$, when n is 0, are identical or different and represent halogen, phenyl or a group of the formulae (III), (IV) or (V)

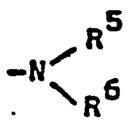

(III),

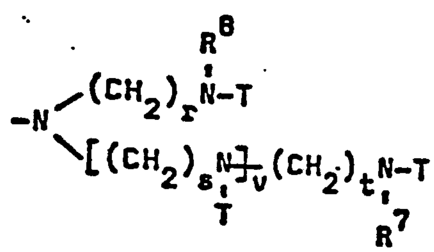

(IV)

$-OR^9$ (V)

but when n is 1, $X_2$ is always a group of the formula

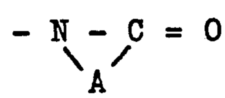

with A being $C_4$ to $C_{11}$ alkylene, wherein, in the formulae (III), (IV) and (V), $R^5$ and $R^6$ are identical or different and represent radicals listed for $R^2$ and $R^6$ additionally has the meaning of $C_3$ to $C_{21}$ alkoxyalkyl, or $R^5$ and $R^6$, together with the N atom connecting them, also represent a pyrrolidine ring or a piperidine, morpholine or hexamethyleneimine ring which is unsubstituted or substituted by up to four $C_1$ to $C_4$ alkyl groups, r, s, and t are identical or different whole numbers from 2 to 6 and v is from 0 to 3, $R^7$ and $R^8$ represent identical of different radicals with the meanings indicated for $R^2$, T has the meaning of a group of the formula (VI)

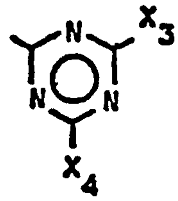

(VI)

with $X_3$ and $X_4$ being identical or different radicals of the formulae (III), (IV) or (V), $R^9$ is one of the radicals mentioned under $R^1$, when m is 0, and Y represents $C_2$ to $C_{18}$ alkylene, $C_2$ to $C_{12}$ bis(propyloxy)alkylene, monocycloalkylene, dicycloalkylene or tricycloalkylene having 6 to 18 C atoms, which can be substituted by up to four methyl groups and in which up to two C atoms can be replaced by N atoms which can carry propylene groups, or Y represents $C_6$ to $C_{18}$ arylene, or $C_7$ to $C_{14}$ aralkylene, or a piperazinylene radical can also take the place of the group

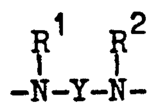

,

or $C_5$ to $C_9$ azamonocycloalkylene or azadicycloalkylene radicals, substituted by up to four methyl groups, can take the place of the group

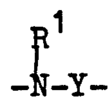

or

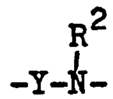

,

and in formula (I), at least one group of the formula (II) is present, preferably at least one group of the formula (II) per triazine ring, which comprises reacting a compound of the formula (VII)

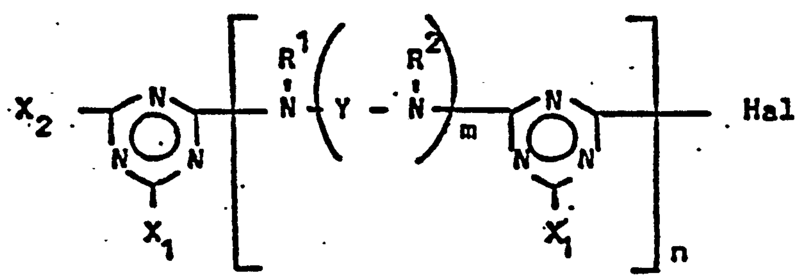

(VII)

in which $R^1$, $R^2$, $X_1$, $X_2$, Y, m and n have the meanings indicated above and Hal is preferably chlorine, in an inert organic solvent at 100 to 200°C with 1.0 to 1.05 times the equivalent amount, relative to the halogen atoms envisaged for the reaction, of the alkali metal salt of a lactam having 4 to 11 methylene groups.

2. The use of a compound as claimed in claim 1 for stabilizing synthetic polymers.

3. The use as claimed in claim 2, wherein the polymer is a polyolefin, polyacrylate, polymethacrylate or a homopolymer or copolymer of styrene.

4. A process for stabilizing synthetic polymers against the damaging effects of light, wherein 0.01 to 5 parts by weight, relative to polymer, of a stabilizer as claimed in claim 1 are added to polymers, optionally in addition to substances having a stabilizing action which are already known.

5. A synthetic polymer, stabilized against UV decomposition, containing 0.01 to 5 parts by weight, relative to polymer, of a stabilizer as claimed in claim 1.

**Revendications**

pour les Etats contractants: BE, CH, DE, FR, GB, IT, LI, NL, SE.

1. Composés répondant à la formule générale (I):

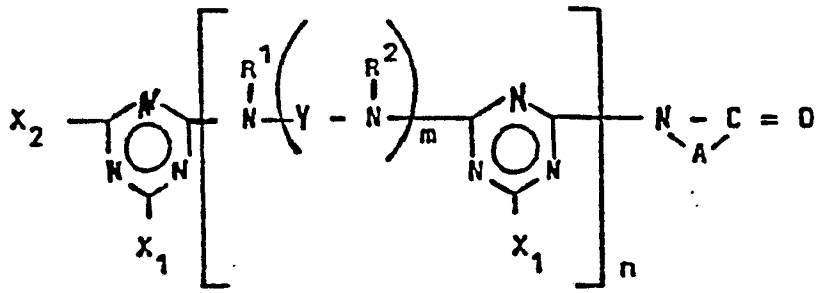

(I)

dans laquelle:
m vaut 0 ou 1,
n vaut également 0 ou 1, et
A représente un groupe alkylène en $C_4$-$C_{11}$,
$R^1$ représente, quand m est nul, un atome d'hydrogène, un groupe alkyle en $C_1$-$C_{18}$, un groupe alcoxyalkyle en $C_3$-$C_{21}$, un groupe cycloalkyle en $C_5$-$C_{12}$, qui peut être substitué par un groupe alkyle en $C_1$-$C_4$, un groupe phényle ou naphtyle, qui peut être substitué par jusqu'à deux restes Cl ou alkyle en $C_1$-$C_4$ ou par un groupe alcoxy (en $C_1$-$C_4$) ou carbalcoxy (en $C_1$ ou $C_2$), un groupe phénylalkyle en $C_7$-$C_{14}$ ou un groupe de formule (II):

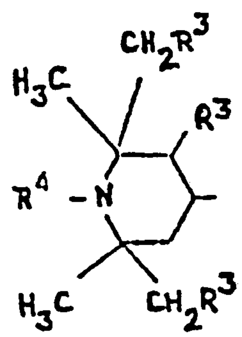

(II)

dans laquelle:
$R^3$ représente un atome d'hydrogène ou un groupe méthyle, et
$R^4$ représente un atome d'hydrogène, un groupe alkyle en $C_1$-$C_4$, époxy-2,3 propyle, allyle ou benzyle,
$R^1$ et $R^2$ sont, quand m vaut 1, identiques ou différents et ils représentent chacun un atome d'hydrogène, un groupe alkyle en $C_1$-$C_{18}$, un groupe cycloalkyle en $C_5$-$C_{12}$ pouvant porter jusqu'à quatre groupes alkyles en $C_1$-$C_4$ comme substituants, un groupe phényle qui peut être substitué par jusqu'à deux restes alkyle en $C_1$-$C_4$, un groupe phénylalkyle en $C_7$-$C_{14}$ ou un groupe de formule (II),
$X_1$ et $X_2$ sont, quand n est nul, identiques ou différents et ils représentent chacun un atome d'halogène, un groupe phényle ou un groupe répondant aux formules (III), (IV) ou (V):

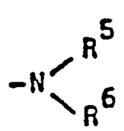

(III),

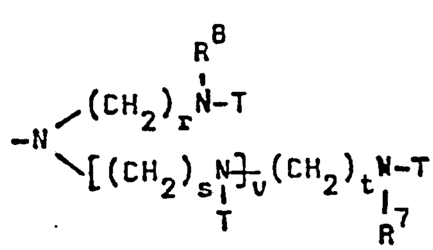

(IV)

$-OR^9$ (V)

mais, quand n vaut 1, $X_2$ représente exclusivement un groupe de formule:

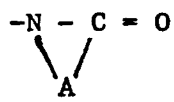

dans laquelle A représente un groupe alkylène en $C_4$-$C_{11}$ et, dans les formules (III), (IV) et (V), $R^5$ et $R^6$ représentent des restes, identiques ou différents, indiqués pour $R^2$, et

$R^6$ peut représenter en outre un groupe alkoxyalkyle en $C_3$-$C_{21}$ ou bien,

$R^5$ et $R^6$, quand ils sont pris ensemble avec l'atome d'azote auquel ils sont liés peuvent représenter un noyau pyrrolidine ou un noyau pipéridine, morpholine ou hexaméthylèneimine non substitué ou pouvant porter jusqu'à quatre groupes alkyle en $C_1$-$C_4$ comme substituants,

r, s et t sont des nombres entiers, identiques ou différents, valant 2 à 6, et

v vaut 0 à 3,

$R^7$ et $R^8$ sont des restes identiques ou différents ayant les sens indiqués pour $R^2$,

T représente un groupe de formule (VI):

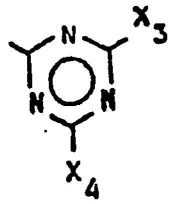

(VI)

dans laquelle:

$X_3$ et $X_4$ sont des restes, identiques ou différents, répondant aux formules (III), (IV), ou (V),

$R^9$ est l'un des restes indiqués pour $R^1$ quand m est nul, et représente un groupe alkylène en $C_2$-$C_{18}$, un groupe bis-(propoxy)-alkylène en $C_2$-$C_{12}$, un groupe mono cycloalkylène, dicycloalkylène ou tricycloalkylène ayant 6 à 18 atomes de carbone et qui peut porter jusqu'à quatre groupes méthyle comme substituants et dans lequel jusqu'à deux atomes de carbone peuvent être remplacés par des atomes d'azote pouvant porter des groupes propylène, un groupe arylène en $C_6$-$C_{18}$ ou un groupe aralkylène en $C_7$-$C_{14}$, ou bien au lieu du groupement

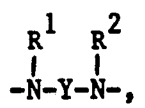

il peut y avoir un reste pipérazinylène ou, au lieu des groupements

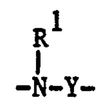

ou

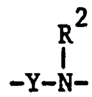

il peut y avoir des restes aza-monocycloalkylène ou aza-dicycloalkylène en $C_5$-$C_9$, pouvant porter jusqu'à quatre groupes méthyle comme substituants, et, dans la formule (I), il y a au moins un groupe de formule (II), et, de préférence, au moins un tel groupe par noyau triazine.

2. Procédé pour préparer des composés selon la revendication 1, caractérisé en ce qu'on fait reagir un composé de formule (VII):

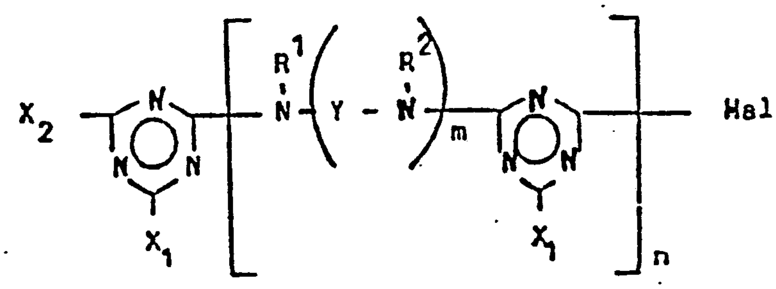 (VII)

dans laquelle $R^1$, $R^2$, $X_1$, $X_2$, Y, m et n ont les sens indiqués à la revendication 1, et Hal représente avantageusement le chlore, dans un solvant organique inerte à 100 jusqu'à 200°C avec la quantité 1,0 fois à 1,05 fois équivalente, par rapport aux atomes d'halogène destinés à la réaction, du sel de métal alcalin d'un lactame comportant 4 à 11 groupes méthylène.

3. Utilisation des composés selon la revendication 1 pour stabiliser des polymères synthétiques.

4. Utilisation selon la revendication 3, caractérisée en ce que le polymère est une polyoléfine, un polyacrylate, un polyméthacrylate ou un homopolymère ou copolymère du styrène.

5. Procédé pour stabiliser des polymères synthétiques contre l'influence nuisible de la lumière, caractérisé en ce qu'on ajoute aux polymères, éventuellement en plus de substances à action stabilisante connues jusqu'à présent, 0,01 à 5 parties en poids, par rapport aux polymères, d'un stabilisant selon la revendication 1.

6. Polymères synthétiques, stabilisés à l'encontre d'une décomposition sous l'effet de l'ultraviolet, et qui contiennent 0,01 à 5 parties en poids, par rapport aux polymères, d'un stabilisant selon la revendication 1.

**Revendications**

pour l'Etat contractant: AT.

1. Procédé pour préparer des triazinyllactames de formule générale (I)

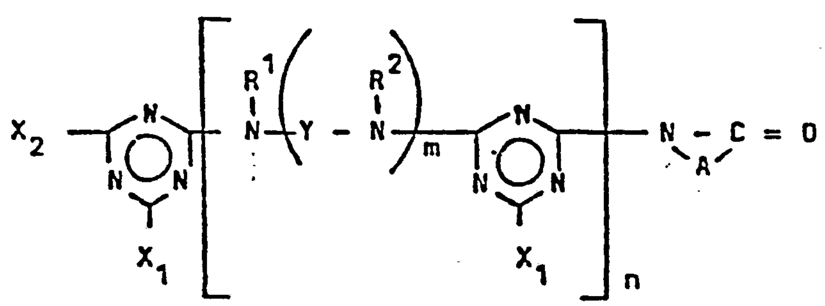 (I)

dans laquelle:

m vaut 0 ou 1,

n vaut également 0 ou 1, et

A représente un groupe alkylène en $C_4$-$C_{11}$,

$R^1$ représente, quand m est nul, un atome d'hydrogène, un groupe alkyle en $C_1$-$C_{18}$, un groupe alcoxyalkyle en $C_3$-$C_{21}$, un groupe cycloalkyle en $C_5$-$C_{12}$, qui peut être substitué par un groupe alkyle en $C_1$-$C_4$, un groupe phényle ou naphtyle, qui peut être substitué par jusqu'à deux restes Cl ou alkyle en $C_1$-$C_4$ ou par un groupe alcoxy (en $C_1$-$C_4$) ou carbalcoxy (en $C_1$ ou $C_2$), un groupe phénylalkyle en $C_7$-$C_{14}$ ou un groupe de formule (II):

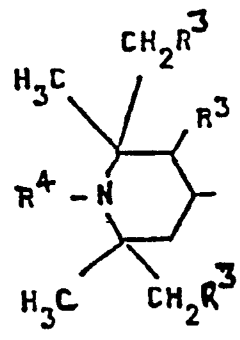

(II)

dans laquelle:

$R^3$ représente un atome d'hydrogène ou un groupe méthyle, et

$R^4$ représente un atome d'hydrogène, un groupe alkyle en $C_1$-$C_4$, époxy-2,3 propyle, allyle ou benzyle,

$R^1$ et $R^2$ sont, quand m vaut 1, identiques ou différents et ils représentent chacun un atome d'hydrogène, un groupe alkyle en $C_1$-$C_{18}$, un groupe cycloalkyle en $C_5$-$C_{12}$ pouvant porter jusqu'à quatre groupes alkyle en $C_1$-$C_4$ comme substituants, un groupe phényle qui peut être substitué par jusqu'à deux restes alkyle en $C_1$-$C_4$, un groupe phénylalkyle en $C_7$-$C_{14}$ ou un groupe de formule (II),

$X_1$ et $X_2$ sont, quand n est nul, identiques ou différents et ils représentent chacun un atome d'halogène, un groupe phényle ou un groupe répondant aux formules (III), (IV) ou (V):

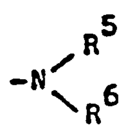

(III),

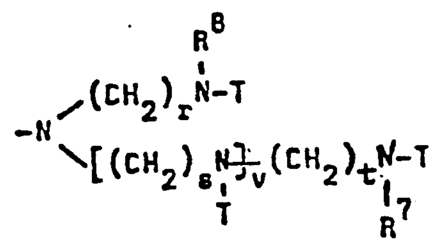

(IV)

$-OR^9$ (V)

mais, quand n vaut 1, $X_2$ représente exclusivement un groupe de formule:

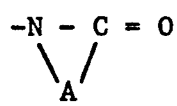

dans laquelle A représente un groupe alkylène en $C_4$-$C_{11}$ et, dans les formules (III), (IV) et (V),

$R^5$ et $R^6$ représentent des restes, identiques ou différents, indiqués pour $R^2$, et

$R^6$ peut représenter en outre un groupe alcoxyalkyle en $C_3$-$C_{21}$ ou bien,

$R^5$ et $R^6$, quand ils sont pris ensemble avec l'atome d'azote auquel ils sont liés, peuvent représenter un noyau pyrrolidine ou un noyau pipéridine, morpholine ou hexaméthylèneimine non substitué ou pouvant porter jusqu'à quatre groupes alkyle en $C_1$-$C_4$ comme substituants,

r, s et t sont des nombres entiers, identiques ou différents, valant 2 à 6, et

v vaut 0 à 3,

$R^7$ et $R^8$ sont des restes identiques ou différents ayant les sens indiques pour $R^2$,

T représent un groupe de formule (VI):

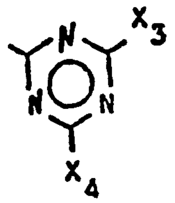 (VI)

dans laquelle:

$X_3$ et $X_4$ sont des restes, identiques ou différents, répondant aux formules (III), (IV), ou (V),

$R^9$ est l'un des restes indiqués pour $R^1$ quand m est nul, et

Y représente un groupe alkylène en $C_2$-$C_{18}$, un groupe bis-(propoxy)-alkylène en $C_2$-$C_{12}$, un groupe monocycloalkylène, dicycloalkylène ou tricycloalkylène ayant 6 à 18 atomes de carbone, pouvant porter jusqu'à quatre groupes méthyle comme substituants et dans lequel jusqu'à deux atomes de carbone peuvent être remplacés par des atomes d'azote pouvant porter des groupes propylène, un groupe arylène en $C_6$-$C_{18}$ ou un groupe aralkylène en $C_7$-$C_{14}$, ou bien au lieu du groupement

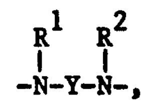

il peut y avoir un reste pipérazinylène ou, au lieu des groupements

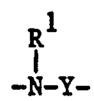

ou

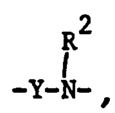

il peut y avoir des restes aza-monocycloalkylène ou aza-dicycloaklylène en $C_5$-$C_9$, pouvant porter jusqu'à quatre groupes méthyle comme substituants, et, dans la formule (I), il y a au moins un groupe de formule (II), et, de préférence, au moins un tel groupe par noyau triazine, procédé caractérisé en ce qu'on fait réagir un composé de formule (VII):

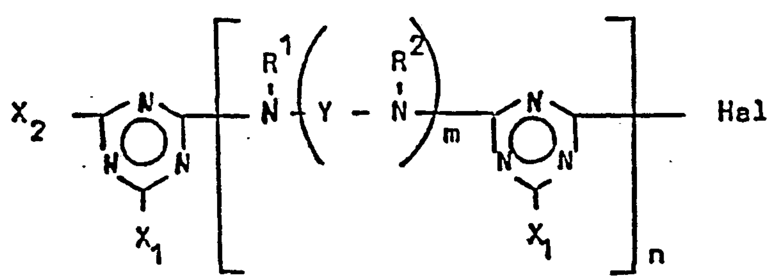 (VII)

(dans laquelle $R^1$, $R^2$, $X_1$, $X_2$, Y, m et n ont les sens indiqués ci-dessus et Hal représente de préférence un atome de chlore) dans un solvant organique inerte à 100 jusqu'à 200°C avec la quantité représentant 1,0 à 1,05 fois la quantité équivalente, par rapport aux atomes d'halogène destinés à la réaction, du sel de métal alcalin d'un lactame comportant 4 à 11 groupes méthylène.

2. Utilisation des composés selon la revendication 1 pour stabiliser des polymères synthétiques.

3. Utilisation selon la revendication 2, caractérisée en ce que le polymère est une polyoléfine, un polyacrylate, un polyméthacrylate ou un homopolymère ou copolymère du styrène.

4. Procédé pour stabiliser des polymères synthétiques contre l'influence nuisible de la lumière, caractérisé en ce qu'on ajoute aux polymères, éventuellement en plus de substances à action stabilisante connues jusqu'à

présent, 0,01 à 5 parties en poids, par rapport aux polymères, d'un stabilisant selon la revendication 1.

5. Polymères synthétiques, stabilisés à l'encontre d'une décomposition sous l'effet de l'ultraviolet, et qui contiennent 0,01 à 5 parties en poids, par rapport aux polymères, d'un stabilisant selon la revendication 1.